# EUROPÄISCHE PATENTSCHRIFT

(11) **EP 0 558 952 B1**
(45) Veröffentlichungstag und Bekanntmachung des Hinweises auf die Patenterteilung: **27.12.1996**
(21) Anmeldenummer: 93101799.0
(22) Anmeldetag: 05.02.1993
(51) Int. Cl.: C07D 301/14, C07D 301/19, C07D 303/04, C07D 303/06, C07D 303/08

(54) **Fluorsubstituierte Epoxycyclo- und Epoxybicyclo(2.2.1)Heptan-Derivate sowie Verfahren zur Herstellung**
Fluorosubstituted epoxycyclohexanes and epoxybicyclo(2.2.1)-heptanes and process for their preparation
Dérivés d'époxycyclohexane et d'époxybicyclo(2.2.1)heptane fluorés et procédé pour leur préparation

(30) Priorität: 29.02.1992 DE 4206387
(43) Veröffentlichungstag der Anmeldung: 08.09.1993
(73) Patentinhaber: HOECHST AKTIENGESELLSCHAFT, 65926 Frankfurt am Main (DE)
(72) Erfinder: Kulpe, Jürgen, Dr., W-6230 Frankfurt/Main 80 (DE); Strutz, Heinz, Dr., W-6230 Frankfurt/Main 80 (DE)

(56) Entgegenhaltungen:
- US-A- 3 238 227
- US-A- 3 631 072
- JOURNAL OF THE AMERICAN CHEMICAL SOCIETY, Band 77, 1955, Gaston, PA (US); Seiten 915-919
- JOURNAL OF ORGANIC CHEMISTRY, Band 38, Nr. 11, 1973, Easton, PA (US); Seiten 2027-2042
- JOURNAL OF NEUROCHEMISTRY, Band 57, Nr. 2, 1991; Seiten 509-519
- METHODEN DER ORGANISCHEN CHEMIE, Houben-Weyl, Band VI/3, Sauerstoffverbindungen I, 1965, Georg Thieme Verlag, Stuttgart (DE); Seiten 385-402

## Beschreibung

Die vorliegende Erfindung betrifft fluorsubstituierte Epoxide der allgemeinen Formel 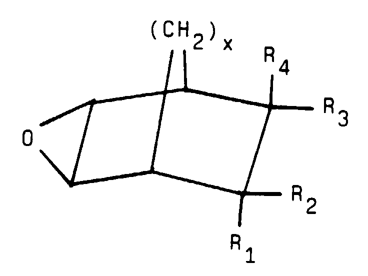 worin die Substituenten R₁ bis R₄ unabhängig voneinander folgende Bedeutung haben:
R₁ bis R₄ = H, Fluor, C₁- bis C₁₈-Alkyl,
bei dem ein Teil oder alle Wasserstoffe durch Fluor substituiert sein können, und mindestens einer der Substituenten R₁ bis R₄ ein vollständig fluoriertes Alkyl oder ein Alkyl der Formel

   -(CH₂)ₘ-(CₙF₂ₙ₊₁),

   in der m für 1 oder 2 und n für eine ganze Zahl von 1 bis 17 steht, ist, und x für 0 oder 1 steht,
sowie ein Verfahren zu ihrer Herstellung.

Aus der großen Auswahl möglicher fluorsubstituierter Epoxide sind folgende Verbindungen von besonderem Interesse:
a) 2,3-Epoxy-5,5-bis(trifluormethyl)bicyclo[2.2.1]heptan, 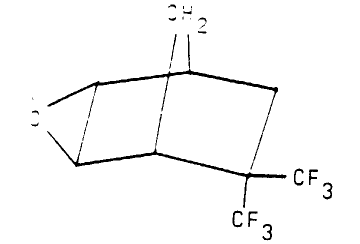
b) 2,3-Epoxy-5,6,6-trifluor-5-(trifluormethyl)bicyclo[2.2.1]-heptan 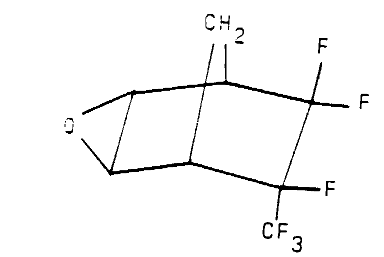
c) 2,3-Epoxy-5-(perfluorhexyl)bicyclo[2.2.1]heptan 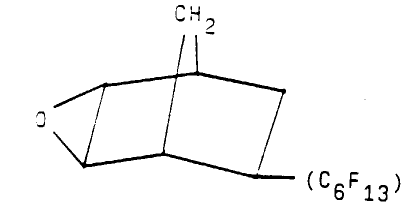
d) 2,3-Epoxy-5-(perfluoroctyl)bicyclo[2.2.1]heptan 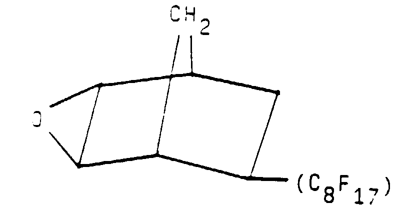
e) 1,2-Epoxy-4-(perfluorhexyl)cyclohexan 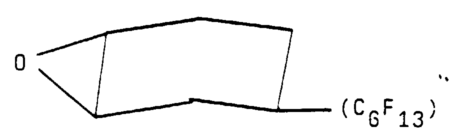
f) 2,3-Epoxy-5-(2,2,3,3,4,4,5,5,6,6,7,7,7-tridecafluorheptyl)bicyclo[2.2.1.]heptan 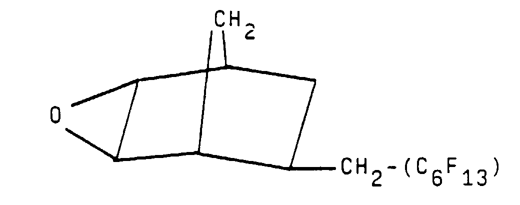
g) 2,3-Epoxy-5-(3,3,4,4,5,5,6,6,7,7,8,8,8-tridecafluoroctyl)bicyclo[2,2,1]heptan

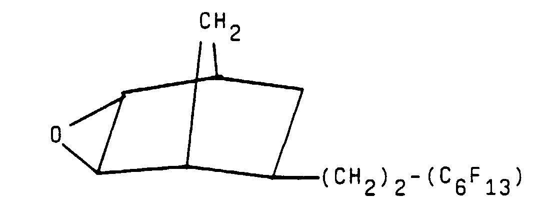

Als Ausgangsprodukte für die Herstellung fluorsubstituierter Epoxide wird auf das Umsetzungsprodukt von Butadien oder Cyclopentadien mit fluorsubstituiertem Alken zurückgegriffen. Die Umsetzung von Butadien bzw. Cyclopentadien mit Olefinen wird in der Literatur als "Diels-Alder-Reaktion" beschrieben. Im J. Am. Chem. Soc. (1955) Vol. 77 Pag. 915 - 919 und J. Org. Chem. (1973) Vol 38 No. 11 Pag. 2027 - 2042 sind Verfahren zur Herstellung von Bicyclo[2.2.1]hepten-Derivaten beschrieben, welche Fluor enthalten und als Einsatzstoffe für die Herstellung der fluorsubstituierten Epoxide geeignet sind.

Das erfindungsgemäße Verfahren zur Herstellung fluorsubstituierter Epoxide aus dem Umsetzungsprodukt von Butadien oder Cyclopentadien mit fluorsubstituiertem Alken nach der Arbeitsweise einer Diels-Alder-Reaktion ist dadurch gekennzeichnet, daß man das Umsetzungsprodukt in trockenem organischem Lösemittel löst und in diese Lösung bei Normaltemperatur eine 1- bis 3-fache äquivalente Menge eines organischen Peroxides, bezogen auf das Umsetzungsprodukt, zugibt und bei Temperaturen zwischen 40 und 120°C über einen Zeitraum von 5 bis 50 Stunden reagieren läßt, danach im Vakuum die Leichtsieder abdestilliert und anschließend das fluorsubstituierte Epoxid reinigt.

Das erfindungsgemäße Verfahren zur Herstellung fluorsubstituierter Epoxide kann wahlweise und bevorzugt dadurch gekennzeichnet sein, daß man
1) als organisches Peroxid Peroxyessigsäure oder t-Butylhydroperoxid mit Molybdänylacetylacetonat als Katalysator zugibt,
2) als trockenes organisches Lösemittel Methylenchlorid, Benzol, Xylol oder Toluol in einer Menge von 100 bis 1000 g, bezogen auf 100 g Umsetzungsprodukt, verwendet,
3) das organische Peroxid als 0,2 bis 5 molare organische Lösung zugibt,
4) das fluorsubstituierte Epoxid nach Entfernen der Leichtsieder über eine Vakuumdestillation reinigt,
5) das fluorsubstituierte Epoxid nach Entfernen der Leichtsieder an Kieselgel chromatographiert und als Laufmittel ein Gemisch aus Hexan/Methylenchlorid/Diethylether (10/10/0.5 Volumenteile) verwendet.

Die erfindungsgemäßen fluorsubstituierten Epoxide können zur Herstellung von Polyethern oder Polyglykolen eingesetzt werden.

Molybdänylacetylacetonat wurde von der Firma Aldrich Chemie GmbH & Co. KG, 7924 Steinheim bezogen.

Bei der gewählten Herstellungsart des Diels-Alder-Umsetzungsproduktes entsteht ein Isomerengemisch aus exo- und endo-Additionsprodukten. Es können aber auch die reinen exo- oder endo-Additionsprodukte einer Diels-Alder-Reaktion als Ausgangsprodukt für die Herstellung fluorsubstituierter Epoxide eingesetzt werden.

### Beispiel A (Diels-Alder-Reaktion)

### (Diels-Alder-Umsetzungsprodukt)

In einem 1 l Autoklav werden 554 ml = 884 g (2,5 mol) Perfluorhexylethen, 165 g (2,5 mol) frisch destilliertes Cyclopentadien und 5 g Hydrochinon vorgelegt. Der Autoklav wird zweimal mit Stickstoff gespült. Anschließend werden 5 bar Stickstoff aufgedrückt und der Autoklav auf 170°C erwärmt. Nach 72 Stunden läßt man abkühlen, entspannt den Stickstoffüberdruck und filtriert den Autoklaveninhalt in eine Destillationsvorlage. Bei der fraktionierenden Vakuumdestillation werden 783 g des Diels-Alder-Produktes mit einem Siedepunkt von 33 - 35°C bei einem Druck von 0,25 Torr isoliert.

Durch ¹H-NMR-Spektroskopie wurde ein Endo/ Exo-Isomerenverhältnis von 77/23 bestimmt.

Die nachfolgenden Beispiele sollen die Herstellung der erfindungsgemäßen Verbindungen erläutern.

### Beispiel 1

### Herstellung von 2,3-Epoxy-5,5-bis(trifluormethyl)bi-cyclo[2.2.1]heptan

11,5 g (50 mmol) des Diels-Alder-Umsetzungsproduktes von 3,3,3-Trifluor-2-trifluormethyl-1-propen und Cyclopentadien werden in 10 ml trockenem Toluol mit 326 mg (1 mmol) Molybdänylacetylacetonat vermischt und bei 22°C 21 ml einer 2,9 molaren Lösung von t-Butylhydroperoxid in Toluol (61 mmol) innerhalb von 30 Minuten zugetropft. Dabei steigt die Temperatur auf 45°C an. Nun wird die Lösung auf 60°C erwärmt und nach zweieinhalb Stunden 3 g Molekularsieb 4 A sowie weitere 5 ml der 2,9 molaren Lösung von t-Butylhydroperoxid in Toluol zugegeben. Nach 20 Stunden Reaktionszeit werden das Lösungsmittel, t-Butanol sowie noch vorhandenes t-Butylhydroperoxid vorsichtig im Vakuum abgezogen. Der Rückstand wird an 160 g Kieselgel chromatographiert. Als Laufmittel dient ein Gemisch von Hexan/Methylenchlorid/Diethylether (10/10/0,5 Volumenteile). Die produkthaltigen Fraktionen werden vereinigt und im Vakuum fraktionierend destilliert. Das Produkt geht bei einem Druck von 150 - 190 mbar und einer Temperatur von 100 - 124°C über.
Ausbeute: 7,94 g = 64 % der Theorie

| Elementaranalyse: | | | |
|---|---|---|---|
| | C | H | F |
| ber. | 43,9 | 3,3 | 46,3 |
| gef. | 44,5 | 3,4 | 45,5 |

### Beispiel 2

### Herstellung von 2,3-Epoxy-5,6,6-trifluor-5-(trifluormethyl)bicyclo[2.2.1]heptan

8,6 g (40 mmol) des Diels-Alder-Umsetzungsproduktes von Hexafluorpropen und Cyclopentadien werden in 20 ml trockenem Methylenchlorid mit 97 ml (80 mmol) einer 0,83 molaren Lösung von Peroxyessigsäure in Methylenchlorid vermischt und 96 Stunden bei Raumtemperatur gerührt. Anschließend werden die Leichtsieder im Vakuum abdestilliert. Der Rückstand wird zweimal mit je 20 ml Wasser gewaschen und die organische Phase mit Magnesiumsulfat getrocknet. Das Produkt geht bei einem Druck von 100 mbar und einem Siedepunkt von 60 - 65°C über. Zur Erhöhung der Produktreinheit wird die Destillation wiederholt.
Ausbeute: 2,81 g = 30 % der Theorie
Die Identifizierung erfolgt durch Gaschromatographie mit massenselektiver Detektion: M⁺ = 232.

### Beispiel 3

### Herstellung von 2,3-Epoxy-5-(perfluorhexyl)bicyclo[2.2.1]-heptan

103 g (250 mmol) des Diels-Alder-Umsetzungsproduktes von 3,3,4,4,5,5,6,6,7,7,8,8,8-Tridecafluor-1-octen (Perfluorhexylethen) und Cyclopentadien werden in 10 ml trockenem Toluol mit 815 mg (2,5 mmol) Molybdänylacetylacetonat vermischt und bei 25°C 104 ml einer 3,6 molaren Lösung von t-Butylhydroperoxid in Toluol (375 mmol) innerhalb von 50 Minuten zugetropft. Dabei steigt die Temperatur auf 45°C an. Dann wird die Lösung 24 Stunden bei 60°C gehalten und danach das Lösungsmittel, t-Butanol sowie noch vorhandenes t-Butylhydroperoxid im Rotationsverdampfer im Vakuum abdestilliert. Der Rückstand wird über Kieselgel filtriert und zweimal mit je 10 ml Toluol nachgewaschen. Bei der Destillation über eine 15 cm lange Füllkörperkolonne, welche mit 4 mm Glasringen gefüllt ist, geht das Produkt bei einem Druck von 350 mbar und einem Siedepunkt von 54 - 56°C über. Ausbeute: 70,0 g = 65 % der Theorie

### Beispiel 4

### Herstellung von 2,3-Epoxy-5-(perfluoroctyl)bicyclo[2.2.1] heptan

128 g (250 mmol) des Diels-Alder-Umsetzungsproduktes von 3,3,4,4,5,5,6,6,7,7,8,8,9,9,10,10,10-Heptadecafluor-1-decen (Perfluoroctylethen) und Cyclopentadien werden in 10 ml trockenem Toluol mit 815 mg (2,5 mmol) Molybdänylacetylacetonat vermischt und bei 28°C 104 ml einer 3,6 molaren Lösung von t-Butylhydroperoxid in Toluol (375 mmol) innerhalb von 2 Stunden zugetropft. Dabei steigt die Temperatur auf 40°C. Nun wird die Lösung 14 Stunden auf 65°C und danach weitere 2 Stunden auf 80°C gehalten. Das Reaktionsgemisch wird über Magnesiumsilikat filtriert und zweimal mit je 20 ml Toluol nachgewaschen. Die Leichtsieder werden am Rotationsverdampfer abgezogen. Der erhaltene Rückstand wird mit 50 ml Hexan aufgenommen und 12 Stunden bei -28°C der Kristallisation überlassen. Der erhaltene Feststoffbrei wird abgesaugt und im Ölpumpenvakuum bei Raumtemperatur getrocknet.
Ausbeute: 96,7 g = 73,2 % der Theorie
Schmelzpunkt: 72 - 74°C

| Elementaranalyse: | | | |
|---|---|---|---|
| | C | H | F |
| ber. | 34,11 | 1,72 | 61,15 |
| gef. | 33,9 | 1,8 | 61,2 |

### Beispiel 5

### Herstellung von 2,3-Epoxy-5-(2,2,3,3,4,4,5,5,6,6,7,7,7-tridecafluorheptan)bicyclo[2.2.1]heptan

1o,7 g (25 mmol) des Diels-Alder-Produktes von 4,4,5,5,6,6,7,7,8,8,9,9,9-Tridecafluor-1-nonen (Perfluorhexylpropen-1) und Cyclopentadien werden in 10 ml trockenem Toluol mit 326 mg (1 mmol) Molybdänylacetylacetonat vermischt und bei 22°C 13 ml einer 2,9 molaren Lösung von t-8utylhydroperoxid in Toluol (37,5 mmol) innerhalb von 30 Minuten zugetropft. Dabei erhöht sich die Temperatur auf 60°C. Die Lösung wird dann weitere 2 Stunden bei 60°C gehalten. Die Leichtsieder werden im Vakuum bei 40°C abdestilliert und der Rückstand an Kieselgel chromatographiert. Als Laufmittel dient ein Gemisch von Hexan/Methylenchlorid/ Diethylether (10/10/0,5 Volumenteile). Die produkthaltigen Fraktionen werden vereinigt. Nach Abdestillieren des Laufmittels verbleiben 7,3 g eines weißen Feststoffes.
Ausbeute: 7,3 g = 66 % der Theorie
Schmelzpungt: 39 - 41°C

| Elementaranalyse: | | | |
|---|---|---|---|
| | C | H | F |
| ber. | 38,93 | 2,51 | 55,85 |
| gef. | 38,3 | 2,6 | 55,1 |

### Beispiel 6

### Herstellung von 2,3-Epoxy-5-(3,3,4,4,5,5,6,6,7,7,8,8,8-tridecafluoroctan)bicyclo[2.2.1]heptan

4,4 g (10 mmol) des Diels-Alder-Proudktes von 5,5,6,6,7,7,8,8,9,9,10,10,10-Tridecafluor-1-decen (Perfluorhexylbuten-1) und Cyclopentadien werden in 10 ml trockenem Toluol mit 133 mg (0,4 mmol) Molybdänylacetylacetonat vermischt und bei 22°C 5,2 ml einer 2,9 molaren Lösung von t-Butylhydroperoxid in Toluol (1,5 mmol) innerhalb von 15 Minuten zugetropft. Dabei steigt die Temperatur auf 31°C. Anschließend wird die Lösung 17 Stunden auf 60°C gehalten. Das Lösungsmittel wird dann am Rotationsverdampfer im Vakuum bei Raumtemperatur entfernt. Der Rückstand wird an Kieselgel chromatographiert. Als Laufmittel dient ein Gemisch von Hexan/Methylenchlorid/Diethylether (10/10/0,5 Volumenteile). Die produkthaltigen Fraktionen werden vereinigt. Nach Abdestillieren des Laufmittels verbleiben 2,0 g eines weißen Feststoffes.
Ausbeute = 43 % der Theorie.

## Patentansprüche (Patentansprüche für folgende(n) Vertragsstaat(en): AT, BE, CH, DE, FR, GB, IT, LI, NL)

1. Fluorsubstituierte Epoxide der allgemeinen Formel 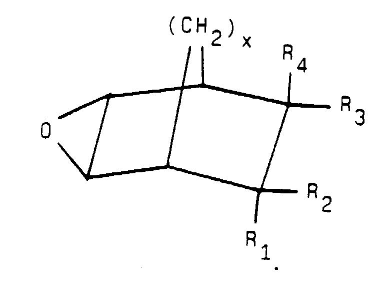 worin die Substituenten R₁ bis R₄ unabhängig voneinander folgende Bedeutung haben:
R₁ bis R₄ = H, Fluor, C₁- bis C₁₈-Alkyl,
bei dem ein Teil oder alle Wasserstoffe durch Fluor substituiert sein können, und mindestens einer der Substituenten R₁ bis R₄ ein vollständig fluoriertes Alkyl oder ein Alkyl der Formel
-(CH₂)ₘ-(CₙF₂ₙ₊₁),
in der m für 1 oder 2 und n für eine ganze Zahl von 1 bis 17 steht, ist und x für 0 oder 1 steht.

2. Das fluorsubstituierte Epoxid nach Anspruch 1, dadurch gekennzeichnet, daß es 2,3-Epoxy-5,5-bis(trifluormethyl)bicyclo[2.2.1]heptan,

3. Das fluorsubstituierte Epoxid nach Anspruch 1, dadurch gekennzeichnet, daß es 2,3-Epoxy-5,6,6-trifluor-5-(trifluormethyl)bicyclo[2.2.1]heptan ist.

4. Das fluorsubstituierte Epoxid nach Anspruch 1, dadurch gekennzeichnet, daß es 2,3-Epoxy-5-(perfluorhexyl)bicyclo[2.2.1]heptan ist.

5. Das fluorsubstituierte Epoxid nach Anspruch 1, dadurch gekennzeichnet, daß es 2,3-Epoxy-5-(perfluoroctyl)bicyclo[2.2.1]heptan ist.

6. Das fluorsubstituierte Epoxid nach Anspruch 1, dadurch gekennzeichnet, daß es 1,2-Epoxy-4-(perfluorhexyl)cyclohexan ist.

7. Das fluorsubstituierte Epoxid nach Anspruch 1, dadurch gekennzeichnet, daß es 2,3-Epoxy-5-(2,2,3,3,4,4,5,5,6,6,7,7,7-tridecafluorheptyl)bicyclo[2.2.1]heptan ist.

8. Das fluorsubstituierte Epoxid nach Anspruch 1, dadurch gekennzeichnet, daß es 2,3-Epoxy-5-(3,3,4,4,5,5,6,6,7,7,8,8,8-tridecafluoroctyl)bicyclo [2.2.1]heptan ist.

9. Verfahren zur Herstellung fluorsubstituierter Epoxide, nach einem der Ansprüche 1 bis 8, aus dem Umsetzungsprodukt von Butadien oder Cyclopentadien mit fluorsubstituiertem Alken nach der Arbeitsweise einer Diels-Alder-Reaktion, dadurch gekennzeichnet, daß man das Umsetzungsprodukt in trockenem organischem Lösemittel löst und in diese Lösung bei Normaltemperatur eine 1- bis 3-fache äquivalente Menge eines organischen Peroxides, bezogen auf das Umsetzungsprodukt, zugibt und bei Temperaturen zwischen 4o und 12o °C über einen Zeitraum von 5 bis 5o Stunden reagieren läßt, danach im Vakuum die Leichtsieder abdestilliert und anschließend das fluorsubstituierte Epoxid reinigt.

10. Verfahren nach Anspruch 9, dadurch gekennzeichnet, daß man als organisches Peroxid Peroxyessigsäure oder t-Butylhydroperoxid mit Molybdänylacetylacetonat als Katalysator zugibt.

11. Verfahren nach einem der Ansprüche 9 oder 10, dadurch gekennzeichnet, daß man als trockenes organisches Lösemittel Methylenchlorid, Benzol, Xylol oder Toluol in einer Menge von 1oo bis 1ooo g, bezogen auf 1oo g Umsetzungsprodukt, verwendet.

12. Verfahren nach einem der Ansprüche 9 bis 11, dadurch gekennzeichnet, daß man das organische Peroxid als 0,2 bis 5 molare organische Lösung zugibt.

13. Verfahren nach einem der Ansprüche 9 bis 12, dadurch gekennzeichnet, daß man das fluorsubstituierte Epoxid nach Entfernen der Leichtsieder über eine Vakuumdestillation reinigt.

14. Verfahren nach einem der Ansprüche 9 bis 12, dadurch gekennzeichnet, daß man das fluorsubstituierte Epoxid nach Entfernen der Leichtsieder an Kieselgel chromatographiert und als Laufmittel ein Gemisch aus Hexan/Methylenchlorid/Diethylether (1o/1o/o.5 Volumenteile) verwendet.

## Patentansprüche (Patentansprüche für folgende(n) Vertragsstaat(en): ES)

1. Verfahren zur Herstellung fluorsubstituierter Epoxide der allgemeinen Formel 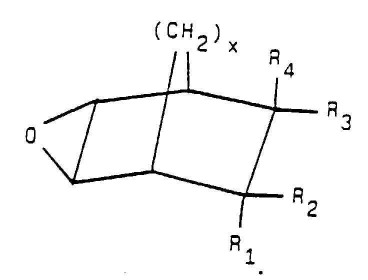 worin die Substituenten R₁ bis R₄ unabhängig voneinander folgende Bedeutung haben:
R₁ bis R₄ = H, Fluor, C₁- bis C₁₈-Alkyl,
bei dem ein Teil oder alle Wasserstoffe durch Fluor substituiert sein können, und mindestens einer der Substituenten R₁ bis R₄ ein vollständig fluoriertes Alkyl oder ein Alkyl der Formel
-(CH₂)ₘ-(CₙF₂ₙ₊₁),
in der m für 1 oder 2 und n für eine ganze Zahl von 1 bis 17 steht, ist und x für 0 oder 1 steht, aus dem Umsetzungsprodukt von Butadien oder Cyclopentadien mit fluorsubstituiertem Alken nach der Arbeitsweise einer Diels-Adler-Reaktion, dadurch gekennzeichnet, daß man das Umsetzungsprodukt in trocknem organischem Lösemittel löst und in diese Lösung bei Normaltemperatur eine 1- bis 3-fache äquivalente Menge eines organischen Peroxids, bezogen auf das Umsetzungsprodukt, zugibt und bei Temperaturen zwischen 40 und 120 °C über einen Zeitraum von 5 bis 50 Stunden reagieren läßt, danach im Vakuum die Leichtsieder abdestilliert und anschließend das fluorsubstituierte Epoxid reinigt.

2. Verfahren nach Anspruch 1, dadurch gekennzeichnet, daß man als organisches Peroxid Peroxyessigsäure oder t-Butylhydroperoxid mit Molybdänylacetylacetonat als Katalysator zugibt.

3. Verfahren nach einem der Ansprüche 1 oder 2, dadurch gekennzeichnet, daß man als trockenes organisches Lösemittel Methylenchlorid, Benzol, Xylol oder Toluol in einer Menge von 1oo bis 1ooo g, bezogen auf 1oo g Umsetzungsprodukt, verwendet.

4. Verfahren nach einem der Ansprüche 1 bis 3, dadurch gekennzeichnet, daß man das organische Peroxid als 0,2 bis 5 molare organische Lösung zugibt.

5. Verfahren nach einem der Ansprüche 1 bis 4, dadurch gekennzeichnet, daß man das fluorsubstituierte Epoxid nach Entfernen der Leichtsieder über eine Vakuumdestillation reinigt.

6. Verfahren nach einem der Ansprüche 1 bis 4, dadurch gekennzeichnet, daß man das fluorsubstituierte Epoxid nach Entfernen der Leichtsieder an Kieselgel chromatographiert und als Laufmittel ein Gemisch aus Hexan/Methylenchlorid/Diethylether (1o/1o/o.5 Volumenteile) verwendet.

## Claims (Claims for the following Contracting State(s): AT, BE, CH, DE, FR, GB, IT, LI, NL)

1. A fluorine-substituted epoxide of the formula 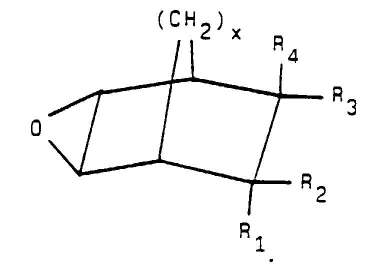 in which the substituents R₁ to R₄, independently of one another, have the following meanings:
R₁ to R₄ = H, fluorine, C₁- to C₁₈-alkyl
in which some or all of the hydrogens may be substituted by fluorine, and at least one of the substituents R₁ to R₄ is a completely fluorinated alkyl or an alkyl of the formula
-(CH₂)ₘ-(CₙF₂ₙ₊₁),
in which m is 1 or 2, and n is an integer from 1 to 17, and x is 0 or 1.

2. A fluorine-substituted epoxide as claimed in claim 1, being 2,3-epoxy-5,5-bis(trifluoromethyl)bicyclo[2.2.1]heptane.

3. A fluorine-substituted epoxide as claimed in claim 1, being 2,3-epoxy-5,6,6-trifluoro-5-(trifluoromethyl)bi-cyclo[2.2.1]heptane.

4. A fluorine-substituted epoxide as claimed in claim 1, being 2,3-epoxy-5-(perfluorohexyl)bicyclo[2.2.1]heptane.

5. A fluorine-substituted epoxide as claimed in claim 1, being 2,3-epoxy-5-(perfluorooctyl)bicyclo[2.2.1]heptane.

6. A fluorine-substituted epoxide as claimed in claim 1, being 1,2-epoxy-4-(perfluorohexyl)cyclohexane.

7. A fluorine-substituted epoxide as claimed in claim 1, being 2,3-epoxy-5-(2,2,3,3,4,4,5,5,6,6,7,7,7-tridecafluoroheptyl)bicyclo[2.2.1]heptane.

8. A fluorine-substituted epoxide as claimed in claim 1, being 2,3-epoxy-5-(3,3,4,4,5,5,6,6,7,7,8,8,8-tridecafluorooctyl)bicyclo[2.2.1]heptane.

9. A method for the preparation of a fluorine-substituted epoxide as claimed in any one of claims 1 to 8, from the reaction product of butadiene or cyclopentadiene with a fluorine-substituted alkene according to the procedure of a Diels-Alder reaction, which comprises dissolving the reaction product in dry organic solvent, adding to this solution at normal temperature an organic peroxide in an amount from 1 equivalent to 3 times the equivalent, based on the reaction product, reacting the mixture at temperatures between 40 and 120°C over an interval from 5 to 50 hours, then distilling off the low-boiling components under reduced pressure, and subsequently purifying the fluorine-substituted epoxide.

10. The method as claimed in claim 9, wherein the organic peroxide added is peroxyacetic acid or t-butyl hydroperoxide, with molybdenum acetylacetonate as a catalyst.

11. The method as claimed in either of claims 9 and 10, wherein the dry organic solvent used is methylene chloride, benzene, xylene or toluene, in an amount from 100 to 1000 g, based on 100 g of reaction product.

12. The method as claimed in any one of claims 9 to 11, wherein the organic peroxide is added as a 0.2 to 5 molar organic solution.

13. The method as claimed in any one of claims 9 to 12, wherein the fluorine-substituted epoxide is purified, after removal of the low-boiling components, by vacuum distillation.

14. The method as claimed in any one of claims 9 to 12, wherein the fluorine-substituted epoxide, after removal of the low-boiling components, is chromatographed on silica gel, and the mobile solvent used is a mixture of hexane/methylene chloride/diethyl ether (10:10:0.5 parts by volume).

## Claims (Claims for the following Contracting State(s): ES)

1. A method for the prepartion of a fluorine-substituted epoxide of the formula 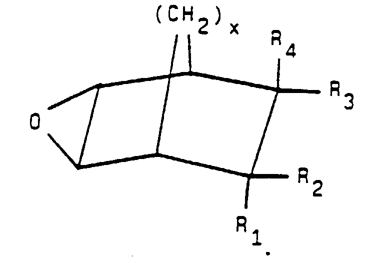 in which the substituents R₁ to R₄, independently of one another, have the following meanings:
R₁ to R₄ = H, fluorine, C₁- to C₁₈-alkyl
in which some or all of the hydrogens may be substituted by fluorine, and at least one of the substituents R₁ to R₄ is a completely fluorinated alkyl or an alkyl of the formula
-(CH₂)ₘ-(CₙF₂ₙ₊₁),
in which m is 1 or 2, and n is an integer from 1 to 17, and x is 0 or 1, from the reaction product of butadiene or cyclopentadiene with a fluorine-substituted alkene according to the procedure of a Diels-Alder reaction, which comprises dissolving the reaction product in dry organic solvent, adding to this solution at normal temperature an organic peroxide in an amount from 1 equivalent to 3 times the equivalent, based on the reaction product, reacting the mixture at temperatures between 40 and 120°C over an interval from 5 to 50 hours, then distilling off the low-boiling components under reduced pressure, and subsequently purifying the fluorine-substituted epoxide.

2. The method as claimed in claim 1, wherein the organic peroxide added is peroxyacetic acid or t-butyl hydroperoxide, with molybdenum acetylacetonate as a catalyst.

3. The method as claimed in either of claims 1 or 2, wherein the dry organic solvent used is methylene chloride, benzene, xylene or toluene, in an amount from 100 to 1000 g, based on 100 g of reaction product.

4. The method as claimed in any one of claims 1 to 3, wherein the organic peroxide is added as a 0.2 to 5 molar organic solution.

5. The method as claimed in any one of claims 1 to 4, wherein the fluorine-substituted epoxide is purified, after removal of the low-boiling components, by vacuum distillation.

6. The method as claimed in any one of claims 1 to 4, wherein the fluorine-substituted epoxide, after removal of the low-boiling components, is chromatographed on silica gel, and the mobile solvent used is a mixture of hexane/methylene chloride/diethyl ether (10:10:0.5 parts by volume).

## Revendications (Revendications pour l'(les) Etat(s) contractant(s) suivant(s): AT, BE, CH, DE, FR, GB, IT, LI, NL)

1. Epoxydes à substituants fluoro répondant à la formule générale : 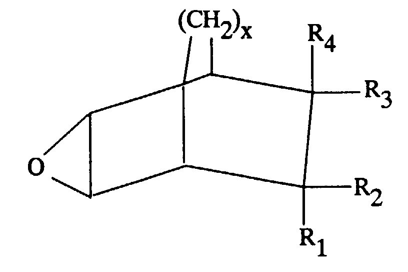 dans laquelle les symboles R₁ à R₄ représentent chacun, indépendamment les uns des autres, l'hydrogène, le fluor, un groupe alkyle en C₁-C₁₈ dans lequel les atomes d'hydrogène peuvent être remplacés en totalité ou en partie par le fluor, et au moins un des symboles R₁ à R₄ représente un groupe alkyle entièrement fluoré ou un groupe alkyle de formule
-(CH₂)ₘ-(CₙF₂ₙ₊₁),
dans laquelle m est égal à 1 ou 2 et n est un nombre entier allant de 1 à 17, et x est égal à 0 ou 1.

2. L'époxyde à substituants fluoro selon revendication 1, caractérisé en ce qu'il consiste en
le 2,3-époxy-5,5-bis(trifluorométhyl)bicydo[2.2.1]heptane.

3. L'époxyde à substituants fluoro selon revendication 1, caractérisé en ce qu'il consiste en
le 2,3-époxy-5,6,6-trifluoro-5-(trifluorométhyl)bicyclo[2.2.1]heptane.

4. L'époxyde à substituants fluoro selon revendication 1, caractérisé en ce qu'il consiste en
le 2,3-époxy-5-(perfluorhexyl)bicyclo[2.2.1]heptane.

5. L'époxyde à substituants fluoro selon revendication 1, caractérisé en ce qu'il consiste en
le 2,3-époxy-5-(perfluoroctyl)bicyclo[2.2.1]heptane.

6. L'époxyde à substituants fluoro selon revendication 1, caractérisé en ce qu'il consiste en
le 1,2-époxy-4-(perfluorhexyl)cyclohexane.

7. L'époxyde à substituants fluoro selon revendication 1, caractérisé en ce qu'il consiste en
le 2,3-époxy-5-(2,2,3,3,4,4,5,5,6,6,7,7,7-tridécafluorheptyl)-bicyclo[2.2,1]heptane.

8. L'époxyde à substituants fluoro selon revendication 1, caractérisé en ce qu'il consiste en
le 2,3-époxy-5-(3,3,4,4,5,5,6,6,7,7,8,8,8-tridécafluoroctyl)bicyclo[2.2.1]heptane.

9. Procédé de préparation des époxydes à substituants fluoro selon une des revendications 1 à 8, à partir du produit de la réaction de Diels-Alder entre le butadiène ou le cyclopentadiène et un alcène à substituants fluoro, caractérisé en ce que l'on dissout le produit de réaction dans un solvant organique sec, on ajoute à cette solution, à température normale, un peroxyde organique en quantité de 1 à 3 équivalents par rapport au produit de réaction et on laisse réagir à des températures de 40 à 120°C pendant des durées de 5 à 50 h puis on distille les fractions volatiles sous vide et on purifie l'époxyde à substituants fluoro.

10. Procédé selon revendication 9, caractérisé en ce que l'on utilise en tant que peroxyde organique l'acide peroxyacétique ou l'hydroperoxyde de tert-butyle avec l'acétylacétonate de molybdényle en tant que catalyseur.

11. Procédé selon une des revendications 9 ou 10, caractérisé en ce que l'on utilise en tant que solvant organique sec le chlorure de méthylène, le benzène, le xylène ou le toluène en quantité de 100 à 1 000 g pour 100 g du produit de réaction.

12. Procédé selon une des revendications 9 à 11, caractérisé en ce que l'on introduit le peroxyde organique à l'état de solution organique 0,2 à 5 M.

13. Procédé selon une des revendications 9 à 12, caractérisé en ce que, après élimination des fractions volatiles, on purifie l'époxyde à substituants fluoro par une distillation sous vide.

14. Procédé selon une des revendications 9 à 12, caractérisé en ce que, après élimination des fractions volatiles, on chromatographie l'époxyde à substituants fluoro sur gel de silice en éluant par un mélange hexane/chlorure de méthylène/éther diéthylique, 10:10:0,5 parties en volume.

## Revendications (Revendications pour l'(les) Etat(s) contractant(s) suivant(s): ES)

1. Procédé pour la préparation d'époxydes à substituants fluoro de formule générale : 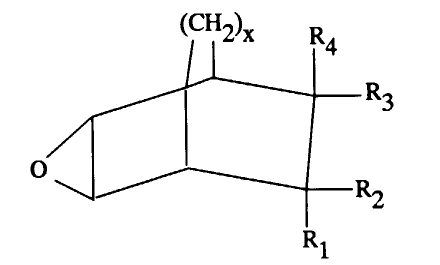 dans laquelle les symboles R₁ à R₄ représentent chacun, indépendamment les uns des autres, l'hydrogène, le fluor, un groupe alkyle en C₁-C₁₈ dans lequel les atomes d'hydrogène peuvent être remplacés en totalité ou en partie par le fluor, et au moins un des symboles R₁ à R₄ représente un groupe alkyle entièrement fluoré ou un groupe alkyle de formule
-(CH₂)ₘ-(CₙF₂ₙ₊₁),
dans laquelle m est égal à 1 ou 2 et n est un nombre entier allant de 1 à 17, et x est égal à 0 ou 1, à partir du produit de la réaction de Diels-Alder entre le butadiène ou le cyclopentadiène et un alcène à substituants fluoro, caractérisé en ce que l'on dissout le produit de réaction dans un solvant organique sec, on ajoute à cette solution, à température normale, un peroxyde organique en quantité de 1 à 3 équivalents par rapport au produit de réaction et on laisse réagir à des températures de 40 à 120°C pendant des durées de 5 à 50 h puis on distille les fractions volatiles sous vide et on purifie l'époxyde à substituants fluoro.

2. Procédé selon revendication 1, caractérisé en ce que l'on utilise en tant que peroxyde organique l'acide peroxyacétique ou l'hydroxyperoxyde de tert-butyle, avec l'acétylacétonate de molybdényle en tant que catalyseur.

3. Procédé selon une des revendications 1 ou 2, caractérisé en ce que l'on utilise en tant que solvant organique sec le chlorure de méthylène, le benzène, le xylène ou le toluène en quantité de 100 à 1 000 g pour 100 g du produit de réaction.

4. Procédé selon l'une des revendications 1 à 3, caractérisé en ce que l'on introduit le peroxyde organique à l'état de solution organique 0,2 à 5M.

5. Procédé selon l'une des revendications 1 à 4, caractérisé en ce que, après élimination des fractions volatiles, on purifie l'époxyde à substituants fluoro par une distillation sous vide.

6. Procédé selon l'une des revendications 1 à 4, caractérisé en ce que, après élimination des fractions volatiles, on chromatographie l'époxyde à substituants fluoro sur gel de silice en éluant par un mélange hexane/chlorure de méthylène/éther diéthylique, 10:10:0,5 parties en volume.
